(19) **Europäisches Patentamt / European Patent Office / Office européen des brevets**

(11) **EP 2 249 667 B1**

(12) **EUROPEAN PATENT SPECIFICATION**

(45) Date of publication and mention
of the grant of the patent:
**16.05.2012 Bulletin 2012/20**

(21) Application number: **09708006.3**

(22) Date of filing: **02.02.2009**

(51) Int Cl.:
*A23L 1/29* (2006.01)  *A23L 1/305* (2006.01)

(86) International application number:
**PCT/EP2009/051153**

(87) International publication number:
**WO 2009/098182 (13.08.2009 Gazette 2009/33)**

(54) **NUTRITIOUS DRINK**

NAHRHAFTES GETRÄNK

BOISSON NUTRITIONNELLE

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **04.02.2008 EP 08151009**

(43) Date of publication of application:
**17.11.2010 Bulletin 2010/46**

(73) Proprietor: **Novozymes A/S
2880 Bagsvaerd (DK)**

(72) Inventors:
• **NIELSEN, Per Munk
DK-2880 Bagsvaerd (DK)**

• **PETTERSSON, Dan
DK-2880 Bagsvaerd (DK)**

(56) References cited:
**EP-A- 1 797 891       WO-A-2004/026048
WO-A-2008/110515    US-A- 5 962 254
US-A1- 2005 202 147**

• **HOPPE C, ANDERSEN GS ET AL: "The use of
whey or skimmed milk powder in fortified blended
foods for vulnerable groups" J. NUTR., vol. 138,
January 2008 (2008-01), pages 145S-161S,
XP002524029**

Printed by Jouve, 75001 PARIS (FR)

**Description**

**TECHNICAL FIELD**

The present invention relates to a nutritious drink for preventing or treating undernourishment, especially in the third world, and to a method for producing such nutritious drink.

**BACKGROUND OF THE INVENTION**

In refugee and other emergency situations, particularly in the third world, food aid programs are often established which strive to eradicate hunger and malnutrition and thereby save lives.

Food aid programs depend on nutritious food products being available at low cost. Hopper et al, Nutz 2008; 138:1615 describe such products and a potential way to improve these. An optimal product for use in food aid programs would be a product which is concentrated and highly nutritious, easily digestible, requires limited cooking, has good sensory quality, has extended shelf-life and is easy to transport, and comprises all nutrients required by the human body including carbohydrate, fat and protein in balanced amounts as well as vitamins and minerals.

One of the objectives for the present inventors was to develop a vegetable-based, nutritious, water soluble powder at low cost price which has an optimum food energy composition.

Hydrolyzed vegetable proteins, such as soy protein hydrolysates, find application as nutrients, e.g. as nutritional additives to foods and beverages. Hydrolyzed proteins are absorbed more easily than unhydrolysed protein, why protein hydrolysates are considered having a high nutritional value. Several methods for preparing vegetable protein hydrolysates for, e.g., animal feed are known (see, e.g., WO95/28850). However, in many cases enzymatic protein hydrolysates of, e.g., soy have an unpleasant taste. One objective of the present invention was to develop a vegetable-based nutritious drink having a pleasant taste.

All cereals contain phytate and phytic acid as a storage phosphorus source. Phytic acid chelates minerals such as calcium, zinc, magnesium and iron, thereby decreasing the bioavailability of nutritionally important minerals. Phytases are enzymes which catalyze the hydrolyzing off of inorganic phosphorous from phytate. Phytases have been obtained from, e.g., *Bacillus, Pseudomonas, Saccharomyces* and *Aspergillus.* It has previously been suggested to add phytase to feed to avoid supplementing the feed with inorganic phosphorus (US 3,297,548), to use phytase to increase the solubility of vegetable proteins (WO 95/28850), or to include phytase in foods and drinks for human consumption to increase the uptake of minerals (WO 02/54881).

**SUMMARY OF THE INVENTION**

The present inventors have found a method for producing a concentrated nutritious drink based on enzymatic processing of cereal products, especially wheat gluten, which facilitates digestibility and also results in improved mineral uptake. The method of the invention provides a drink having a pleasant taste, a high nutritional value, a balanced food energy distribution, and the possibility of balancing all nutrients including micronutrients required by the human body in one serving. Further, such drink can be produced at relatively low cost which allows for its use in for instance food aid programs in the third world.

The present invention therefore in one aspect relates to a method for producing a nutritious drink comprising:

a) treating wheat gluten protein with at least one enzyme having phytase activity and at least one proteolytic enzyme to obtain hydrolyzed wheat gluten protein;
b) adding a carbohydrate source to obtain an aqueous composition comprising hydrolyzed wheat gluten protein and carbohydrate;
c) mixing the aqueous composition of step b) with vegetable oil to obtain a composition having a balanced food energy distribution; and
d) homogenizing the composition of step c) to obtain a nutritious drink;

wherein the addition of carbohydrate in step b) can be performed before, during or after the enzyme treatment in step a).

The invention also covers a basis composition of a nutritious drink comprising fat, protein and carbohydrate in balanced amounts. The basis composition can be adjusted to a specific situation of under/malnourishment, e.g. by increasing the content of protein and/or fat. The basis composition comprises hydrolyzed wheat gluten protein which is easily digestible and has a good taste. Flavour, such as vanilla flavour or strawberry flavour, may also be added to further improve the taste. A drink based on the basis composition is also characterized in comprising a low amount of phytic acid, thus improving the bioavailability of nutritionally important minerals. Further, the basis composition can be supplemented with, e.g., minerals, such as iron and calcium, vitamins, amino acids, such as lysine, and thus become a product covering all nutritional needs.

The present invention therefore in one aspect relates to a vegetable-based nutritious drink for preventing or treating undernourishment, wherein:

a) at least 30% of the food energy is from carbohydrate;
b) at least 10% of the food energy is from fat;
c) at least 10% of the food energy is from protein;
d) at least 5% of the dry matter is hydrolyzed wheat gluten protein having a degree of hydrolysis of more than 3%; and
e) the amount of phytic acid is less than one micromole per g dry matter.

## DETAILED DISCLOSURE OF THE INVENTION

The present invention provides a method for producing a nutritious drink which is useful for treating or preventing malnourishment and/or undernourishment.

The invention provides a method for producing a nutritious drink comprising:

a) treating wheat gluten protein with at least one enzyme having phytase activity and at least one proteolytic enzyme to obtain hydrolyzed wheat gluten protein;
b) adding a carbohydrate source to obtain an aqueous composition comprising hydrolyzed wheat gluten protein and carbohydrate;
c) mixing the aqueous composition of step b) with vegetable oil to obtain a composition having a balanced food energy distribution; and
d) homogenizing the composition of step c) to obtain a nutritious drink;

wherein the addition of carbohydrate in step b) can be performed before, during or after the enzyme treatment in step a).

In one preferred embodiment according to the invention, the drink is for use in food aid programs. Such food aid programs may have been established as a school feeding program or due to, e.g., famine, refugee or other emergency situations.

In another embodiment, the drink is for prevention or treatment of malnourishment and/or undernourishment caused by a medical condition. The drink may thus be useful at, e.g., hospitals.

The method of the invention provides a basis composition of a drink having a high nutritional value and comprising organic nutrients in the form of protein, fat and carbohydrate in balanced amounts.

A drink in the context of the present invention is a food product for human consumption which is in the fluid state of matter, i.e., it has the ability to flow. This may also be described as the ability to take on the shape of the container it fills. A drink according to the invention is to be interpreted broadly to also include drinks having a higher viscosity than, e.g., water, beer, milk, etc. A drink in the context of the invention may mean a food product which is pourable and can be readily swallowed without being chewed. Drying of the drink during production for reconstitution immediately before consumption is an option.

In step a) of the method of the invention, wheat gluten protein is treated with at least one enzyme having phytase activity.

A variety of wheat gluten protein materials may be used in the method of the invention. In general, the wheat gluten protein to be used may be derived from wheat in accordance with methods known in the art. It may be made by washing wheat flour dough with water until all the starch is washed out, leaving insoluble gluten as a gummy mass, which is subject to further processing. The wheat gluten material may, e.g., be wheat gluten concentrate or wheat gluten isolate, also known as wheat protein concentrate or wheat protein isolate. It may have a protein content of more than 60%, such as more than 65%, 70%, 75%, 80%, 85%, or 90%.

In the context of this invention, an enzyme having phytase activity is an enzyme which catalyzes the removal of inorganic phosphorous from various myoinositol phosphates. Phytase enzymes are preferably derived from a microbial source such as bacteria, fungi and yeasts, but may also be of vegetable origin.

In a preferred embodiment, the phytase enzyme is derived from a fungal strain, in particular a strain of *Aspergillus,* e.g *Aspergillus niger, Aspergillus oryzae, Aspergillus ficuum, Aspergillus awamori, Aspergillus nidulans* or *Aspergillus terreus.* Most preferred is a phytase enzyme derived from a strain of *Aspergillus niger* or a strain of *Aspergillus oryzae.*

In another preferred embodiment, the phytase enzyme is derived from a bacterial strain, in particular a strain of *Bacillus* or a strain of *Pseudomonas.* Preferably, the phytase enzyme is derived from a strain of *Bacillus subtilis.*

In yet another preferred embodiment, the phytase enzyme is derived from a yeast, in particular a strain of *Kluveromyces* or a strain of *Saccharomyces.* Preferably, the phytase enzyme is derived from a strain of *Saccharomyces cerevisiae.*

In the context of the invention "an enzyme derived from" encompasses an enzyme naturally produced by the particular strain, either recovered from that strain or encoded by a DNA sequence isolated from this strain and produced in a host organism transformed with said DNA sequence.

Preferably, the amount of phytase used in the method of the invention is from about 2 to about 50000 FYT (as defined below) per kg wheat gluten protein, more preferably from about 50 to about 30000 FYT per kg wheat gluten protein, most preferably from about 100 to about 10000 FYT per kg wheat gluten protein.

One Phytase Unit (FYT) is defined as the amount of enzyme which under standard conditions (i.e. at pH 5.5, 37°C, a substrate concentration of 5.0 mM sodium phytate, and a reaction time of 30 minutes) liberates 1 micromole of phosphate per minute.

In step a) of the method of the invention, wheat gluten protein is also treated with at least one proteolytic enzyme to obtain hydrolyzed wheat gluten protein.

The term proteolytic enzyme as used herein is an enzyme that hydrolyses peptide bonds, i.e. has protease activity. Such enzymes may also be referred to as, e.g., proteases, peptidases, proteinases, or peptide hydrolases.

Preferably, the at least one proteolytic enzyme to be used in the method of the present invention is of the endo-type that act internally in polypeptide chains, also referred to as endopeptidases. Such enzyme having endo-proteolytic activity may be combined with an exopeptidase, or a protease preparation having exopeptidase activity.

The proteolytic enzyme may be a microbial enzyme, preferably a protease derived from a bacterial or a fungal strain, or the proteolytic enzyme may be trypsin or pepsin. In a preferred embodiment, the proteolytic enzyme is a bacterial protease derived from a strain of *Bacillus*, preferably a strain of *Bacillus subtilis* or a strain of *Bacillus licheniformis.* Commercially available *Bacillus* proteases are Alcalase and Neutrase (Novozymes A/S, Denmark). In another preferred embodiment, the proteolytic enzyme is a fungal protease derived from a strain of *Aspergillus,* preferably a strain of *Aspergillus aculeatus*, a strain of *Aspergillus niger*, or a strain of *Aspergillus oryzae.* A commercially available *Aspergillus* protease is Flavourzyme (Novozymes A/S, Denmark).

Preferably, the amount of proteolytic enzyme used in the method of the invention is from about 0.0001 to about 0.5 AU (as defined below) per kg wheat gluten protein, preferably from about 0.001 to about 0.5 AU per kg wheat gluten protein, more preferably from about 0.01 to about 0.1 AU per kg wheat gluten protein.

One Anson Unit (AU) is defined as the amount of enzyme which under standard conditions (i.e. 25°C, pH 7.5 and 10 min. reaction time) digests haemoglobin at an initial rate such that there is liberated per minute an amount of TCA soluble product which gives the same colour with phenol reagent as one milliequivalent of tyrosine.

Preferably, the treatment with proteolytic enzyme results in hydrolysed wheat gluten protein having a degree of hydrolysis (DH) of more than 2%, more preferably more than 3%, more than 4% or more than 5%.

The degree of hydrolysis (DH) expresses the extent of the protein hydrolysis obtained by the method. In the context of the invention, the degree of hydrolysis (DH) is defined as follows:

$$DH = (\text{Number of peptide bonds cleaved / Total number of peptide bonds}) \times 100 \%$$

The skilled person will know how to measure the DH.

The treatment with the at least one enzyme having phytase activity and the at least one proteolytic enzyme may be carried out consecutively or simultaneously. If they are carried out consecutively, the treatment with phytase may be carried out before the protease treatment, or the treatment with protease may be carried out before the phytase treatment. Alternatively, the treatment with at least one proteolytic enzyme is carried out for some time before addition of the enzyme having phytase activity and the incubation is continued now with two different enzymes, perhaps at a different temperature. It may also be that treatment with one or more proteolytic enzymes, e.g. one or more endopeptidases, or a combination of endo- and exopeptidases, is carried out for some time before addition of further enzyme(s) having proteolytic activity is added to the composition. Such consecutive enzyme addition may be due to, e.g., different enzymes having different temperature optimum for enzyme activity. The skilled person will know when to add different enzymes to the composition comprising wheat gluten protein to obtain the optimal enzyme reaction(s).

In a preferred embodiment, the wheat gluten protein is gradually added into an aqueous solution of one or more proteolytic enzyme while high shear mixing is performed. The phytase may be added to the solution prior to the wheat gluten protein or it may be added after. Optionally, after the wheat gluten protein has been hydrolyzed by the protease, the temperature of the composition may be adjusted before addition of the phytase.

In step b) of the method of the invention, a carbohydrate source is added to obtain an aqueous composition comprising hydrolyzed wheat gluten protein and carbohydrate.

In one embodiment, the carbohydrate source is glucose syrup or sugar, such as cane sugar or beet sugar.

In a preferred embodiment, the carbohydrate source is cereal-based, e.g. based on wheat, corn, rice, barley or sorghum. In a more preferred embodiment, the cereal-based carbohydrate source is produced from corn, e.g. corn flour. The cereal-based carbohydrate source may be wheat flour. The cereal-based carbohydrate source may be treated with at least one carbohydrate degrading enzyme, preferably to obtain carbohydrate having an average chain length of less than 40, less than 30, less than 20 or less than 15 carbohydrate monomers.

The at least one carbohydrate degrading enzyme may be any enzyme degrading carbohydrates, such as starch. It may be a glycosidase enzyme (EC 3.2, also known as carbohydrases). Preferably, the carbohydrate degrading enzyme is an amylase, in particular an alpha-amylase or a beta-amylase, a cellulase, in particular an endo-1,4-beta-glucanase (EC 3.2.1.4) or an endo-1,3-beta-glucanase (3.2.1.6), a xylanase, in particular an endo-1,4-beta-glucanase (EC 3.2.1.8) or a xylan-endo-1,3-beta-xylosidase (EC 3.2.1.32), an alpha-galactosidase (EC 3.2.1.22), a polygalacturonase (EC 3.2.1.15, also known as pectinases), a cellulose-1,4-beta-cellobiosidase (EC 3.2.1.91, also known as cellobiohydrolases), an endoglucanase, in particular an endo-1,6-beta-glucanase (EC 3.2.1.75), an endo-1,2-beta-glucanase (EC 3.2.1.71), an endo-1,3-beta-glucanase (EC 3.2.1.39) or an endo-1,3-alpha-glucanase (EC 3.2.1.59).

The at least one carbohydrate degrading enzyme may also be a multi-enzyme complex containing, e.g., a range of carbohydrases. Such multi-enzyme complex may also contain other enzyme activities, e.g. pectinase activity. For example, such multi-enzyme complex could be Viscozyme (Novozymes A/S, Denmark), an enzyme preparation from *Aspergillus aculeatus* containing a wide range of carbohydrases, including arabanase, cellulase, beta-glucanase, hemicellulase and xylanase, and also having pectinolytic activity.

The carbohydrate source may be added before, during or after the enzyme treatment in step a). The carbohydrate source may be added after the treatment with the proteolytic enzyme but before treatment with the enzyme having phytase activity.

In a preferred embodiment, the wheat gluten protein is gradually added into an aqueous solution of proteolytic enzyme while high shear mixing is performed. After the wheat gluten protein has been sufficiently hydrolyzed by the protease so that a decreased viscosity is obtained, a cereal-based carbohydrate is added. A carbohydrate degrading enzyme is added either before or after addition of the carbohydrate source and the composition is held at a temperature which allows for the carbohydrate degrading enzyme reaction to take place. In one embodiment, the carbohydrate degrading enzyme is added before the carbohydrate source, and the carbohydrate source is added slowly or gradually. The phytase may be added to the composition prior to the wheat gluten protein or it may be added after. Also, the phytase may be added prior to addition of the carbohydrate degrading enzyme or it may be added after. Optionally, after the wheat gluten protein has been hydrolyzed by the protease, the temperature of the composition may be adjusted before addition of the carbohydrate degrading enzyme and the phytase. Further, the temperature of the composition may be adjusted after the carbohydrate degrading enzyme has been allowed to act and before addition of the phytase.

In another embodiment, a cereal-based carbohydrate source is treated with at least one carbohydrate degrading enzyme and optionally an enzyme having phytase activity prior to being mixed with the hydrolysed wheat gluten protein.

Preferably, the wheat gluten protein and the carbohydrate source are mixed prior to the phytase treatment.

The enzyme reactions in the method of the present invention are carried out at (a) suitable temperature(s) and for sufficient time to allow each enzyme to react with its substrate, i.e. wheat gluten protein and/or cereal-based carbohydrate. The temperature and time of incubation with each enzyme depends on the types and dosages of enzymes used, and the skilled person will know how to adjust these accordingly.

In a preferred embodiment of the method of the invention, the composition of step b), i.e. the aqueous composition comprising wheat gluten protein, which has been treated with protease and phytase, and carbohydrate, which has optionally been treated with carbohydrate degrading enzyme and phytase, comprises less than 2 micromole phytic acid per g dry matter, preferably less than 1 micromole, less than 0.5 micromole, less than 0.4 micromole, less than 0.3 micromole or less than 0.2 micromole per g dry matter.

In another preferred embodiment of the method of the invention, the aqueous composition after step a) and b) has a dry matter content which is above 25%, preferably above 30%, above 35%, above 40%, above 45% or above 50%.

Inactivation of the enzyme(s) may be carried out by conventional methods for inactivating enzymes, e.g. by heat treatment, i.e. elevating the temperature of the hydrolysis suspension or mixture to a temperature which denatures the enzymes, typically to a temperature of above 85°C.

Enzymes used in the method of the invention may be purified. The term "purified" as used herein covers enzyme protein essentially free from components from the native organism from which it is obtained. The term "purified" also covers enzyme protein essentially free from components from the host organism from which it is produced.

Accordingly, an enzyme may be purified, viz. only minor amounts of other proteins being present. The expression "other proteins" relate in particular to other enzymes. The term "purified" as used herein also refers to removal of other components, particularly other proteins and most particularly other enzymes present in the cell of origin of the enzyme. An enzyme may be "substantially pure", i.e. substantially free from other components from the organism in which it is produced, e.g., a host organism for recombinantly produced enzyme.

However, the enzymes to be used in the method of the invention need not be that pure. For instance, a proteolytic enzyme to be used may e.g. include other enzymes, even other proteases, in which case it could be termed a protease preparation.

In step c) of the method of the invention, the aqueous composition of step b) is mixed with vegetable oil to obtain a composition having a balanced food energy distribution.

Preferably, a nutritious drink produced by the method of the invention has a balanced food energy distribution, wherein:

at least 25%, preferably at least 30%, at least 35%, at least 40%, at least 45%, at least 50% or at least 55% of the food energy is from carbohydrate;
at least 5%, preferably at least 10%, at least 15%, at least 20%, at least 25% or at least 30% of the food energy is from fat; and
at least 5%, preferably at least 10%, at least 15% or at least 20% of the food energy is from protein.

More preferably, a nutritious drink produced by the method of the invention has a balanced food energy distribution, wherein between 40 and 70% of the food energy is from carbohydrate, between 15 and 40% of the food energy is from fat and between 10 and 20% of the food energy is from protein. Even more preferably, a nutritious drink according to the invention has a balanced food energy distribution, wherein between 50 and 60% of the food energy is from carbohydrate, between 25 and 35% of the food energy is from fat and between 10 and 15% of the food energy is from protein.

Preferably, a nutritious drink produced by the method of the invention comprises at least 2.5 kcal, such as at least 3, at least 3.5, at least 4, at least 4.5, at least 5, at least 5.5 or at least 6 kcal per g dry matter

The vegetable oil may be vegetable oil from, e.g., palm, sunflower, soybean, rapeseed, corn, coconut, canola, peanut, cotton seed, rice bran or grape seed, or it may be a mixture of different types of vegetable oil.

In a preferred aspect, the vegetable oil to be applied according to the present invention is oil from palm, sunflower, soybean, or rapeseed.

In step d) of the method of the invention, the composition of step c) is homogenized. Homogenization is performed by any method known in the art.

In a preferred embodiment, a nutritious drink produced by the method of the present invention comprises less than 2 micromole phytic acid per g dry matter, preferably less than 1 micromole, less than 0.5 micromole, less than 0.4 micromole, less than 0.3 micromole or less than 0.2 micromole per g dry matter.

In one embodiment, the method of the invention further comprises addition of one or more of vitamins A, B, C, D or E.

In a preferred embodiment, a nutritious drink produced by the method of the present invention comprises at least 0.5, such as at least 0.6, 0.7, 0.8, 0.9 or 1 microgram vitamin A in the form of retinol equivalents per g dry matter.

In another preferred embodiment, a nutritious drink produced by the method of the present invention comprises at least 50, such as at least 60, 70, 80, 90 or 100 microgram vitamin C in the form of ascorbic acid per g dry matter.

In another preferred embodiment, a nutritious drink produced by the method of the present invention comprises at least 0.005, such as at least 0.006, 0.007, 0.008, 0.009 or 0.01 microgram vitamin D per g dry matter.

In another preferred embodiment, a nutritious drink produced by the method of the present invention comprises at least 8, such as at least 9, 10, 11, 12 or 13, microgram vitamin E in the form of alpha-tocopherol equivalents per g dry matter.

In one embodiment, the method of the invention further comprises addition of minerals, such as iron or calcium.

In a preferred embodiment, a nutritious drink produced by the method of the present invention comprises at least 1, such as at least 1.5, 2, 2.5 or 3 mg calcium per g dry matter.

In another preferred embodiment, a nutritious drink produced by the method of the present invention comprises at least 0.005, such as at least 0.01, 0.015, 0.02, 0.025 or 0.03 mg iron per g dry matter.

In one embodiment, the method of the invention further comprises addition of amino acids, such as lysine, methionine or threonine.

In a preferred embodiment, a nutritious drink produced by the method of the present invention comprises at least 0.05 g lysine per g dry matter.

In one embodiment, the method of the invention further comprises addition of flavour, such as vanilla flavour or strawberry flavour.

In one embodiment, the method of the invention further comprises addition of preservative. Such preservative may be any substance suitable for preservation of food and the skilled person will know which preservative may be useful to add.

In a preferred embodiment, the method of the invention further comprises drying the composition after homogenization in step d) to obtain a powder. Drying may be performed by any method known in the art, such as spray drying or roller drying.

The powder can be reconstituted to a nutritious drink by addition of water.

Thus, in a preferred embodiment, the present invention relates to a method for producing a nutritious drink powder comprising:

a) treating wheat gluten protein with at least one enzyme having phytase activity and at least one proteolytic enzyme to obtain hydrolyzed wheat gluten protein;
b) adding a carbohydrate source to obtain an aqueous composition comprising hydrolyzed wheat gluten protein and carbohydrate;
c) mixing the aqueous composition of step b) with vegetable oil to obtain a composition having a balanced food energy distribution; and
d) homogenizing and drying the composition of step c) to obtain a nutritious drink powder;

wherein the addition of carbohydrate in step b) can be performed before, during or after the enzyme treatment in step a).

The invention also relates to a nutritious drink powder obtained by such method.

The invention further provides a vegetable-based nutritious drink for preventing or treating undernourishment, wherein:

a) at least 30% of the food energy is from carbohydrate;
b) at least 10% of the food energy is from fat;
c) at least 10% of the food energy is from protein;
d) at least 5% of the dry matter is hydrolyzed wheat gluten protein having a degree of hydrolysis of more than 3%; and
e) the amount of phytic acid is less than one micromole per g dry matter.

In a preferred aspect, such nutritious drink comprises at least 0.5, such as at least 0.6, 0.7, 0.8, 0.9 or 1, microgram vitamin A (retinol equivalents) per g dry matter.

In another preferred aspect, such nutritious drink comprises at least 50, such as at least 60, 70, 80, 90 or 100, microgram vitamin C (ascorbic acid) per g dry matter.

In another preferred aspect, such nutritious drink comprises at least 0.005, such as at least 0.006, 0.007, 0.008, 0.009 or 0.01, microgram vitamin D per g dry matter.

In another preferred aspect, such nutritious drink comprises at least 8, such as at least 9, 10, 11, 12 or 13, microgram vitamin E (alpha-tocopherol equivalents) per g dry matter.

In another preferred aspect, such nutritious drink comprises at least 1, such as at least 1.5, 2, 2.5 or 3, mg calcium per g dry matter.

In another preferred aspect, such nutritious drink comprises at least 0.005, such as at least 0.01, 0.015, 0.02, 0.025 or 0.03, mg iron per g dry matter.

In another preferred aspect, such nutritious drink comprises at least 4, such as at least 5, 6, 7, 8, 9 or 10, kcal per g dry matter.

In another preferred aspect, such nutritious drink has been supplemented with amino acids, such as lysine, methionine or threonine. The nutritious drink may, e.g., comprise at least 0.05 g lysine per g dry matter.

In another preferred aspect, such nutritious drink comprises flavour, such as vanilla flavour or strawberry flavour.

In another preferred aspect, such nutritious drink comprises preservative.

The invention further relates to a dried food product which can be dissolved in water to make a nutritious drink as described above.

## EXAMPLE 1

A production of dried wheat drink powder was carried out in pilot plant scale to obtain 16.8 kg dried product. In the experiment was used the combined enzyme system consisting of protease and amylase to demonstrate that it is possible to produce a soluble and stable drink product from insoluble wheat derived components supplying the protein and the carbohydrate components to the drink.

Raw materials were:

6.5 kg vital wheat gluten, 36.4 kg wheat flour, 2.49 kg cocofat, 2.35 kg rapeseed oil, 22 g ascorbic acid, 138 g protease (Protamex 1.5MG, activity 1.5 AU/g, from Novozymes A/S, Denmark), 218 g amylase (BAN 240L from Novozymes A/S, Denmark), 52 L water.

The water was heated in a tank to 55°C, the protease added followed by slowly addition of the gluten. During the addition the mixture was re-circulated through a wet milling device (Fryma MZ130 wet mill) and back to the tank.

5 minutes after all gluten was added, the tank temperature was adjusted to 65°C. The amylase was added followed by slowly addition of the wheat flour. 10 minutes after the flour was added, the re-circulation through the wet milling device was stopped and the mixture was left for reaction in the tank at 65°C for 2½ hour.

The product was flash heat treated at 126-130°C for 3-4 sec. at 640 mbar vacuum.

Ascorbic acid, and the oils were added and the mixture was homogenized at 68°C at 300-320 mbar in a Manzon Gaulin homogenizer. Cooling to 5°C and left overnight at 5°C.

The mixture was dried in a Niro Atomizer spray drier with approx 12 kg water evaporation an hour. Inlet temperature was 185-190°C and outlet temperature was 85°C. When 16.8 kg powder was obtained, the drier was stopped and the rest of the mixture was discharged.

The re-constituted powder was producing a homogeneous sweet tasting product.

## EXAMPLE 2

The purpose is to produce some drink products to demonstrate the concept of a drink made from cereal products, i.e. gluten, wheat flour, vegetable oil and using amylase, protease and phytase to make the drink soluble and reduce the phytic acid content.

The composition is made to contain 50% carbohydrate, 15% protein and 30% oil:

Wheat gluten concentrate 5.7%
Wheat flour 33.2%
Vegetable oil (soy bean oil) 10.2%
Water 50.6%
Vanilla flavour (Vanille Crème Extract 125087, Einar Willumsen Denmark) Dosage 0.5 g/L ready drink.

The water was heated to 65°C and Protamex (Protamex 1.5MG, activity 1.5 AU/g, Novozymes A/S, Denmark, dosage 0.3% of dry matter) was added. The wheat gluten concentrate was added gradually while high shear mixing. When all the gluten had been added, amylase (BAN 240L, Novozymes A/S, Denmark, dosage 0.4% of dry matter) and phytase (Bio Feed Phytase L 2X, Novozymes A/S, Denmark, dosage 750 FYT/kg product) was added followed by flour addition. Reaction 2½ hour at 65°C. Then the temperature was increased to 72°C for 5 min. Oil was added followed by homogenization 250bar at 72°C and cooling. Dilution to 12.5% dry matter for taste testing after addition of vanilla flavour 0.5 g/L. The samples were homogeneous with a sweet pleasant vanilla flavour.

2 samples were taken for documentation of the phytase effect: one before addition of phytase (control sample) and one after inactivation (before oil is added). The control sample contained 0.7431 micromole phytic acid/g and the phytase treated 0.0849 micromole phytic acid/g showing a 9-fold reduction in phytic acid.

## Claims

1. A method for producing a nutritious drink comprising:

   a) treating wheat gluten protein with at least one enzyme having phytase activity and at least one proteolytic enzyme to obtain hydrolyzed wheat gluten protein;
   b) adding a carbohydrate source to obtain an aqueous composition comprising hydrolyzed wheat gluten protein and carbohydrate;
   c) mixing the aqueous composition of step b) with vegetable oil to obtain a composition having a balanced food energy distribution; and
   d) homogenizing the composition of step c) to obtain a nutritious drink;

   wherein the addition of carbohydrate in step b) can be performed before, during or after the enzyme treatment in step a).

2. The method of claim 1, wherein the hydrolyzed wheat gluten protein has a degree of hydrolysis of more than 3%.

3. The method of any of the preceding claims, wherein the carbohydrate source is cereal-based.

4. The method of claim 3, wherein the cereal-based carbohydrate source is treated with at least one carbohydrate degrading enzyme to obtain hydrolysed cereal-based carbohydrate.

5. The method of claim 4, wherein the cereal-based carbohydrate source is further treated with a least one enzyme having phytase activity.

6. The method of any of the preceding claims, wherein step a) comprises gradual addition of the wheat gluten protein into an aqueous solution of proteolytic enzyme.

7. The method of any of the preceding claims, wherein step a) comprises high shear mixing.

8. The method of any of the preceding claims, wherein the dry matter content of the composition of step b) is above 30%.

9. The method of any of the preceding claims, wherein the composition of step b) comprises less than 0.5 micromole phytic acid per g dry matter.

10. The method of any of the preceding claims, further comprising addition of one or more of vitamins A, B, C, D and E and/or one or more of calcium and iron.

11. The method of any of the preceding claims, further comprising drying the composition of step d) to obtain a powder which can be reconstituted to a nutritious drink by addition of water.

12. A nutritious drink obtainable by the method of any of the preceding claims.

13. A nutritious drink powder obtainable by the method of claim 11.

14. A vegetable-based nutritious drink for preventing or treating undernourishment, wherein:

    a) at least 30% of the food energy is from carbohydrate;
    b) at least 10% of the food energy is from fat;
    c) at least 10% of the food energy is from protein;
    d) at least 5% of the dry matter is hydrolyzed wheat gluten protein having a degree of hydrolysis of more than 3%; and
    e) the amount of phytic acid is less than one micromole per g dry matter.

15. A dried food product which can be dissolved in water to make a nutritious drink according to claim 14.

**Patentansprüche**

1. Verfahren zum Herstellen eines nahrhaften Getränks, umfassend:

    a) Behandeln von Weizenglutenprotein mit mindestens einem Enzym mit Phytase-Aktivität und mindestens einem proteolytischen Enzym, um hydrolysiertes Weizenglutenprotein zu erhalten;
    b) Hinzufügen einer Kohlenhydratquelle, um eine wässrige Zusammensetzung zu erhalten, die hydrolysiertes Weizenglutenprotein und Kohlenhydrat umfasst;
    c) Mischen der wässrigen Zusammensetzung von Schritt b) mit Pflanzenöl, um eine Zusammensetzung mit einer ausgewogenen Lebensmittelenergieverteilung zu erhalten; und
    d) Homogenisieren der Zusammensetzung von Schritt c), um ein nahrhaftes Getränk zu erhalten;

wobei die Zugabe von Kohlenhydrat in Schritt b) vor, während oder nach der Enzymbehandlung in Schritt a) durchgeführt werden kann.

2. Verfahren nach Anspruch 1, wobei das hydrolysierte Weizenglutenprotein einen Hydrolysegrad von mehr als 3 % aufweist.

3. Verfahren nach einem beliebigen der vorhergehenden Ansprüche, wobei die Kohlenhydratquelle getreidebasiert ist.

4. Verfahren nach Anspruch 3, wobei die getreidebasierte Kohlenhydratquelle mit mindestens einem Kohlenhydrat abbauenden Enzym behandelt wird, um hydrolysiertes getreidebasiertes Kohlenhydrat zu erhalten.

5. Verfahren nach Anspruch 4, wobei die getreidebasierte Kohlenhydratquelle des Weiteren mit mindestens einem Enzym mit Phytase-Aktivität behandelt wird.

6. Verfahren nach einem beliebigen der vorhergehenden Ansprüche, wobei Schritt a) schrittweise Zugabe von dem Weizenglutenprotein in eine wässrige Lösung von proteolytischem Enzym umfasst.

7. Verfahren nach einem beliebigen der vorhergehenden Ansprüche, wobei Schritt a) starkes Schermischen umfasst.

8. Verfahren nach einem beliebigen der vorhergehenden Ansprüche, wobei der Trockenmassegehalt der Zusammensetzung von Schritt b) mehr als 30 % beträgt.

9. Verfahren nach einem beliebigen der vorhergehenden Ansprüche, wobei die Zusammensetzung von Schritt b) weniger als 0,5 Mikromol Phytinsäure pro g Trockenmasse umfasst.

10. Verfahren nach einem beliebigen der vorhergehenden Ansprüche, des Weiteren umfassend die Zugabe von einem oder mehreren der Vitamine A, B, C, D und E und/oder einem oder mehr von Calcium und Eisen.

11. Verfahren nach einem beliebigen der vorhergehenden Ansprüche, des Weiteren umfassend das Trocknen der Zusammensetzung von Schritt d), um ein Pulver zu erhalten, das durch Zugabe von Wasser zu einem nahrhaften Getränk rekonstituiert werden kann.

12. Nahrhaftes Getränk, erhalten durch das Verfahren gemäß einem beliebigen der vorhergehenden Ansprüche.

13. Nahrhaftes Getränkepulver, erhalten durch das Verfahren gemäß Anspruch 11.

14. Pflanzenbasiertes nahrhaftes Getränk zur Vorbeugung oder Behandlung von Unterernährung, wobei:

> a) mindestens 30 % der Lebensmittelenergie aus Kohlenhydrat stammt;
> b) mindestens 10 % der Lebensmittelenergie aus Fett stammt;
> c) mindestens 10 % der Lebensmittelenergie aus Protein stammt;
> d) mindestens 5 % der Trockenmasse hydrolysiertes Weizenglutenprotein mit einem Hydrolysegrad von mehr als 3 % ist; und
> e) die Menge an Phytinsäure weniger als 1 Mikromol pro g Trockenmasse beträgt.

15. Getrocknetes Lebensmittelprodukt, das in Wasser gelöst werden kann, um ein nahrhaftes Getränk gemäß Anspruch 14 herzustellen.

**Revendications**

1. Procédé de production d'une boisson nutritive comprenant les étapes consistant à :

> a) traiter la protéine de gluten de blé avec au moins une enzyme ayant une activité de phytase et au moins une enzyme protéolytique afin d'obtenir une protéine de gluten de blé hydrolysée ;
> b) ajouter une source d'hydrate de carbone afin d'obtenir une composition aqueuse comprenant la protéine de gluten de blé hydrolysée et l'hydrate de carbone ;
> c) mélanger la composition aqueuse de l'étape b) avec de l'huile végétale pour obtenir une composition présentant une répartition énergétique alimentaire équilibrée ; et
> d) homogénéiser la composition de l'étape c) afin d'obtenir une boisson nutritive ;

dans lequel l'addition de l'hydrate de carbone à l'étape b) peut être réalisée avant, pendant ou après le traitement enzymatique de l'étape a).

2. Procédé selon la revendication 1, dans lequel la protéine de gluten de blé hydrolysée a un degré d'hydrolyse de plus de 3 %.

3. Procédé selon l'une quelconque des revendications précédentes, dans lequel la source d'hydrate de carbone est à base de céréales.

4. Procédé selon la revendication 3, dans lequel la source d'hydrate de carbone à base de céréales est traitée avec au moins une enzyme dégradant l'hydrate de carbone pour obtenir l'hydrate de carbone à base de céréale hydrolysé.

**5.** Procédé selon la revendication 4, dans lequel la source d'hydrate de carbone à base de céréales est en outre traitée avec au moins une enzyme ayant une activité de phytase.

**6.** Procédé selon l'une quelconque des revendications précédentes, dans lequel l'étape a) comprend l'addition graduelle de la protéine de gluten de blé à une solution aqueuse d'enzyme protéolytique.

**7.** Procédé selon l'une quelconque des revendications précédentes, dans lequel l'étape a) comprend un mixage à fort cisaillement.

**8.** Procédé selon l'une quelconque des revendications précédentes, dans lequel la teneur en matière sèche de la composition de l'étape b) est supérieure à 30 %.

**9.** Procédé selon l'une quelconque des revendications précédentes, dans lequel la composition de l'étape b) comprend moins de 0,5 micromole d'acide phytique par gramme de matière sèche.

**10.** Procédé selon l'une quelconque des revendications précédentes, comprenant en outre l'addition d'une ou de plusieurs des vitamines A, B, C, D et E et/ou un ou plusieurs éléments parmi le calcium et le fer.

**11.** Procédé selon l'une quelconque des revendications précédentes, comprenant en outre le séchage de la composition de l'étape d) afin d'obtenir une poudre qui peut être reconstituée pour donner une boisson nutritive par addition d'eau.

**12.** Boisson nutritive pouvant être obtenue par le procédé selon l'une quelconque des revendications précédentes.

**13.** Poudre pour une boisson nutritive pouvant être obtenue par le procédé de la revendication 11.

**14.** Boisson nutritive à base de végétaux permettant d'éviter ou de traiter la dénutrition, dans laquelle :

a) au moins 30 % de l'énergie alimentaire provient de l'hydrate de carbone ;
b) au moins 10 % de l'énergie alimentaire provient de la graisse ;
c) au moins 10 % de l'énergie alimentaire provient de la protéine ;
d) au moins 5 % de la matière sèche est constituée par une protéine de gluten de blé hydrolysée ayant un degré d'hydrolyse de plus de 3 % ; et
e) la quantité d'acide phytique est inférieure à un micromole par gramme de matière sèche.

**15.** Produit alimentaire déshydraté qui peut être dissous dans de l'eau pour constituer une boisson nutritive selon la revendication 14.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9528850 A
- US 3297548 A
- WO 0254881 A

**Non-patent literature cited in the description**

- **HOPPER et al.** *Nutz,* 2008, vol. 138, 1615